# EUROPEAN PATENT APPLICATION

(11) **EP 2 386 525 A1**
(43) Date of publication of application: **16.11.2011**
(21) Application number: 10382121.1
(22) Date of filing: 13.05.2010
(51) Int. Cl.: C03C 3/21, C03C 4/00, C03C 12/00, A61L 27/12, A61L 27/46

(54) **Nanostructured material comprising a biocompatible calcium phosphate glass, sol-gel process for its preparation and medical use thereof**

(71) Applicant: Universitat Politècnica de Catalunya, 08034 Barcelona (ES); Fundació Privada Institut de Bioenginyeria de Catalunya, 08028 Barcelona (ES)
(72) Inventor: Castaño Linares, Oscar, 08028 Barcelona (ES); Navarro Toro, Melba, 08028 Barcelona (ES); Planell Estany, Josep Anton, 08034 Barcelona (ES); Engel López, Elisabeth, 08034 Barcelona (ES); Aguirre Cano, Altor, 08034 Barcelona (ES)
(74) Representative: Ponti Sales, Adelaida

(57) **Abstract**

The present invention relates to a nanostructured material comprising a biocompatible calcium phosphate glass in the system P2O5-CaO-MgO-Y2O-TiO2, wherein Y is K or Na. The preferred composition in mol% is 44.5 P2O5, 44.5 CaO, y Y2O, 11-y TiO2 with y ranging from more than 0 up to 11 The present invention also relates to a process for the preparation of said nanostructured material using the sol-gel method, and its uses as a tuneable control ion release agent with specific target cell signalling, particularly for bone tissue regeneration.

## Description

### Field of invention

The present invention relates to the field of glasses, in particular nanostructured materials comprising phosphate glass.

### Background of the invention

Although glasses are commonly included within the ceramics category, they possess very different structures and properties. Basically, a glass is a non-crystalline or amorphous solid with the atomic arrangement of a liquid but whose atoms cannot move. The ASTM defines glasses as: "The inorganic product of fusion which has cooled to a rigid condition without crystallising"[9]. That is, without creating an ordered lattice or atomic structure. Another definition of glass is an: "amorphous material with no long range order presenting glass transition at its Glass Transition Temperature (Tg)".

Phosphate glasses were developed about 100 years ago by Schott et al [1] for use as achromatic optical elements due to their high transparency to UV light, low dispersion and relatively high refractive indices. These glasses are very hygroscopic and therefore, can be degraded in water. Thus, it turns out that phosphate glasses are unstable for many applications. This high solubility has however, been exploited for biomedical applications. In the last decades, phosphate glasses, and more specifically, calcium phosphate glasses have been widely studied not only because of their chemical composition that can be tailored to resemble that of the mineral phase of bone, but also because of their solubility or biodegradability [4].

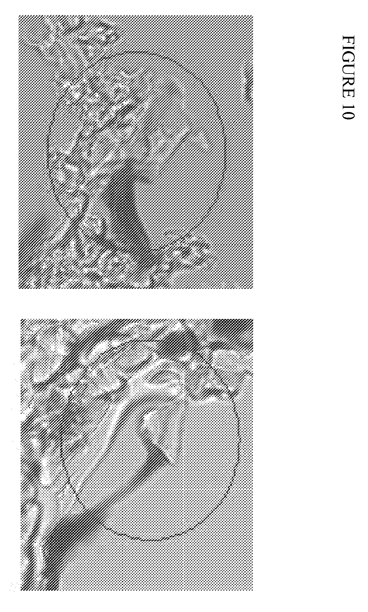

Phosphate-based glasses in the P₂O₅-CaO-Na₂O-TiO₂ system have unique dissolution properties in aqueous-based fluids. Degradation rates can be varied from hours to several weeks by changing the glass composition. Furthermore, these glasses can be synthesised to include modifying oxides that are able to induce a specific biological function and enhance biocompatibility (figure 10) [5, 8].

Calcium phosphate glasses in the system previously mentioned have shown to be biocompatible [5]. Among the potential clinical applications of these glasses are as bone cavity fillers, drug delivery systems, biodegradable reinforcing phase in the case of composites for bone fixation devices and tissue engineering scaffolds. Moreover, phosphate degradable glasses have also been exploited for neural repair, improved haemocompatibility and the controlled release of certain ions. Cu and Ag ions have been used for antibacterial applications [3], fluor release for dental applications; Zn, Cu, and Co as supplements release, and Ca ions for stimulating cell homing and cell proliferation.

Although Si oxide is the most known network former; P₂O₅ is also one of the four classic glass network formers proposed by Zachariesen: SiO₂, GeO₂, B₂O₃ y P₂O₅ [7]. As in the case of silica glasses, phosphate glasses are formed by phosphorus tetrahedral configurations (PO₄). These tetrahedra are linked by covalent bonding between their bridging oxygen and form long phosphate chains, rings and branched polymers which form the glass network.

In general, additionally to the network formers, there are other cations that are called network modifiers. The network modifiers occupy the holes of the network and can have large ionic radii and low valences. If, on the contrary, the network modifiers are small and highly charged cations such as Ti⁴⁺, they tend to cross-link the structure. As previously mentioned, the PO₄ tetrahedron is the basic building block that forms the structure of phosphate-based glasses. Phosphate tetrahedra are classified by the number of oxygen atoms they share with other phosphate tetrahedra. An oxygen atom shared in this way is usually referred to as bridging oxygen. The various types of phosphate tetrahedra that result from this classification are labelled using Qi, where i refers to the number of bridging oxygens and ranges from 0 to 3.

Phosphate glasses may acquire different structures depending on the ratio between modifying oxides and phosphate ions present in the network. Depending on this ratio glasses may have a reticulated network formed by Q₃ groups (corresponding to vitreous P₂O₅), a structure similar to that of polymers with long metaphosphate chains and Q₂ groups, or may be formed by pyrophosphate groups (Q₁) which form very short chains.

Most phosphate-based glasses are prepared by melt-quenching methods. A mixture of oxide precursors is melted in a furnace at temperatures of over 1000°C; the exact temperature used depends on the final composition of the glass. Once a homogeneous melt has been achieved, the glass is formed by casting different shapes as plates. To remove residual stress, the melts are normally annealed at the glass transition temperature (Tg) and cooled very slowly to room temperature.

None of the previous mentioned glasses are nanostructured.

The sol-gel process is a low temperature wet-chemical technique that presents a potential alternative to the melt-quenching method. A typical process starts with a solution of the elements involved in the final product, which reacts with water to produce colloidal particles. This suspension is commonly known as "sol". Typical precursors are inorganic alkoxides and metal chlorides, which undergo hydrolysis and polycondensation reactions to form a colloid. The sol evolves via further condensation reactions towards the formation of an inorganic network containing a liquid phase; this is known as the gel. Growth of an inorganic network occurs via the formation of M-O-M and M-OH-M bridges (where M is an electropositive element, typically Si, Ti, Al or Zr) which generates polymeric species throughout the solvent medium. A drying step serves to remove the liquid phase from the gel thus forming a porous material. Thermal treatment can be used to promote further polycondensation, leading to consolidation and densification of the material structure.

The first sol-gel phosphate-based glasses specifically aimed at biomedical applications were synthesized by Carta et al. [2]. The low temperature of the sol-gel process allows for the production of novel glasses. Moreover, it was shown that this method can be used to produce porous foams, which have potential applications as tissue engineering scaffolds. The versatility of the method allows other promising advantages such as the possibility to introduce biomolecules that are not stable at high temperatures, such as proteins, drugs and polymers during the synthesis process.

Previous works have reported the elaboration of phosphate based glasses by the sol-gel method [6]. However, these glasses have been limited to the ternary system CaO-P₂O₅-TiO₂ without the incorporation of alkali metal oxides such as Na₂O or K₂O. Furthermore, the percentage of TiO₂ in these ternary compositions has not been lower than 25% molar.

An object of the present invention is to provide a material comprising a glass with the formulation P₂O₅:CaO:MgO:Y₂O:TiO₂, wherein Y is K or Na.

Another object of the present invention is to provide a material comprising a glass with the formulation P₂O₅:CaO:MgO:Y₂O:TiO₂, wherein Y is K or Na and the amount of Ti is lower than 25% molar ratio.

A further object of the present invention is to provide a material comprising a glass with the formulation P₂O₅:CaO:MgO:Y₂O:TiO₂, wherein Y is K or Na, which is nanostructured.

A further object of the present invention is to provide a nanostructured material comprising a glass with the formulation P₂O₅:CaO:MgO:Y₂O:TiO₂, wherein Y is K or Na, which is biodegradable and when implanted or administrated, will spontaneously dissolve in 1-12 months.

A further object of the present invention is to provide a nanostructured material comprising a glass with the formulation P₂O₅:CaO:MgO:Y₂O:TiO₂, wherein Y is K or Na, which is useful to stimulate bone and vascular regeneration.

A further object of the invention is to provide an economic and efficient process for obtaining a shape-, size- and polymorph- controlled nanostructured material comprising a glass with the formulation P₂O₅:CaO:MgO:Y₂O:TiO₂, wherein Y is K or Na.

A further object of the invention is to provide a process for obtaining a nanostructured material comprising a glass with the formulation P₂O₅:CaO:MgO:Y₂O:TiO₂, wherein Y is K or Na, in the form of inorganic nanoparticles, composite nanostructured biodegradable fibers, composite nanostructured scaffolds, injectable composite gel, inorganic films or composite films.

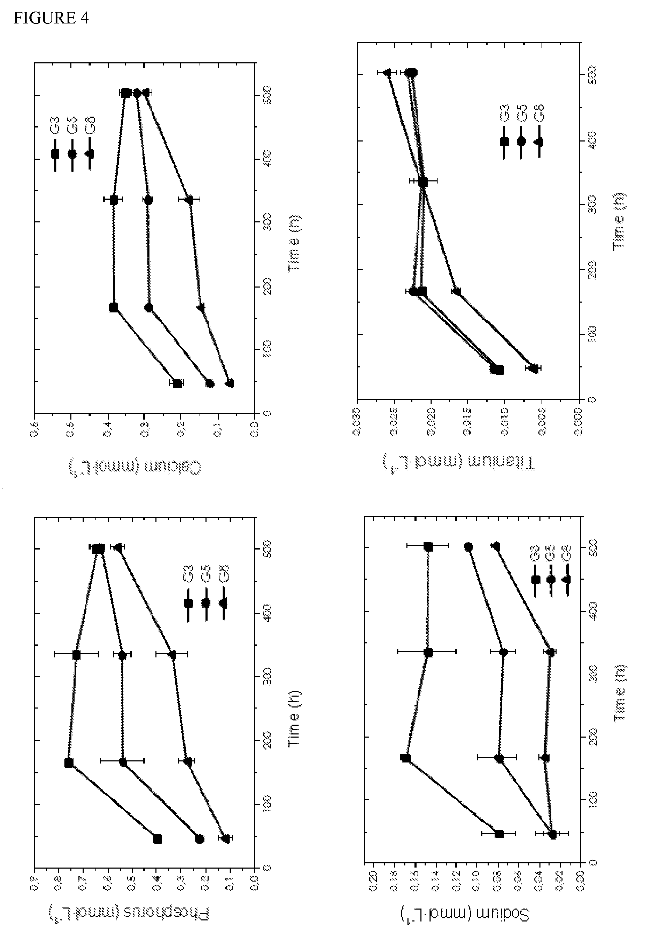

A further object of the invention relates to the use of a nanostructured material comprising a glass with the formulation P₂O₅:CaO:MgO:Y₂O:TiO₂, wherein Y is K or Na, as a tuneable control ion release agent (figure 4).

A further object of the present invention relates to the use of a nanostructured material comprising a glass with the formulation P₂O₅:CaO:MgO:Y₂O:TiO₂, wherein Y is K or Na, for bone or vascular tissue regeneration.

### Summary of the invention

The present invention relates to a nanostructured material comprising a glass with the formulation P₂O₅:CaO:MgO:Y₂O:TiO₂, wherein Y is K or Na.

The present invention also relates to a process for the preparation of a nanostructured material comprising a glass with the formulation P₂O₅:CaO:MgO:Y₂O:TiO₂, wherein Y is K or Na, comprising the steps of:
a) Preparing titanium, phosphorus, potassium or sodium, calcium and magnesium precursor solutions;
b) Preparing a glass precursor solution by mixing in an inert atmosphere the titanium precursor solution; the calcium precursor solution; the magnesium precursor solution; the sodium or potassium precursor solution and the phosphorus precursor solution;
   wherein the molar ratio for P:Ca:Mg:Y:Ti is 89:44,5-x:x:2y:11-y, wherein Y is K or Na, and wherein x is a value equal or higher than 0 and lower than 44,5, and wherein y is a value higher than 0 and lower or equal to 11; and
c) Shaping the precursor mix solution obtained in step b).

The present invention further relates to the use of a nanostructured material comprising a glass with the formulation P₂O₅:CaO:MgO:Y₂O:TiO₂, wherein Y is K or Na, as a tuneable control ion release agent with specific target cell signalling. In particular, the present invention relates to the use for tissue regeneration.

### Brief description of the figures

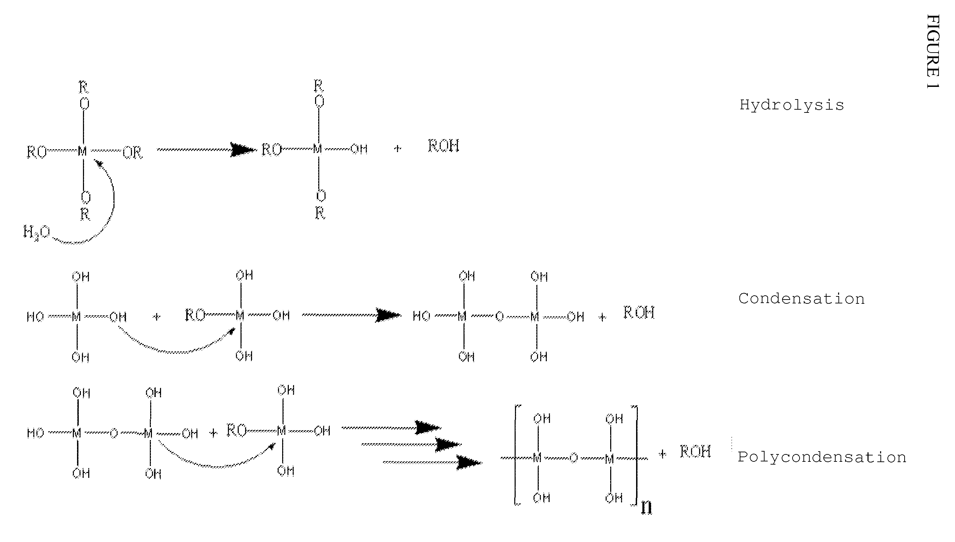

Fig. 1: Exemplary sol-gel reaction mechanism which involves hydrolysis and condensation. Different microstructures and morphologies can be achieved modulating their competition.
Fig. 2: Exemplary scanning electron microscopy of PLA/glass hybrid materials following two different routes for nanoparticles preparation following example 3 (A) and 4 (B).

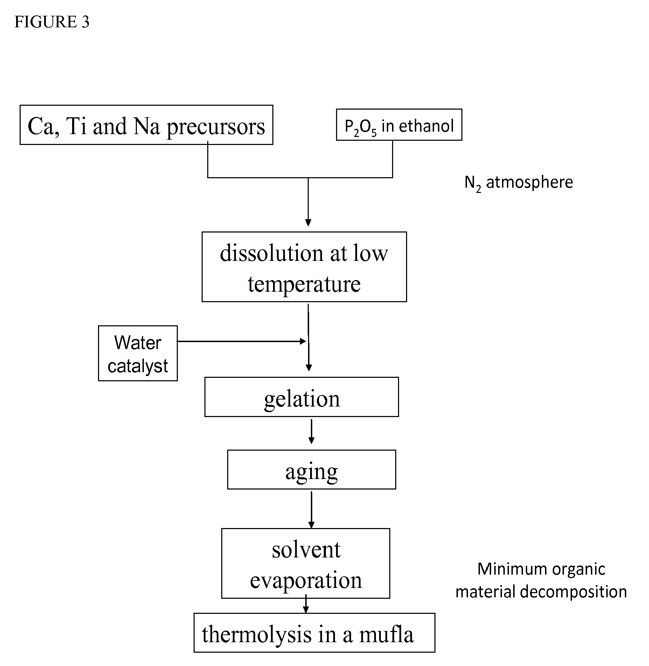

Fig. 3: Exemplary sol-gel experimental process for example 3 nanoparticles preparation.
Figure 4: Evolution of P, Ca, Na and Ti ions release in distilled water with time (500h) for the following phosphate glasses in the system P₂O₅-CaO-Na₂O-TiO₂: G3 (3% TiO₂), G5 (5%TiO₂) and G8 (8% TiO₂).

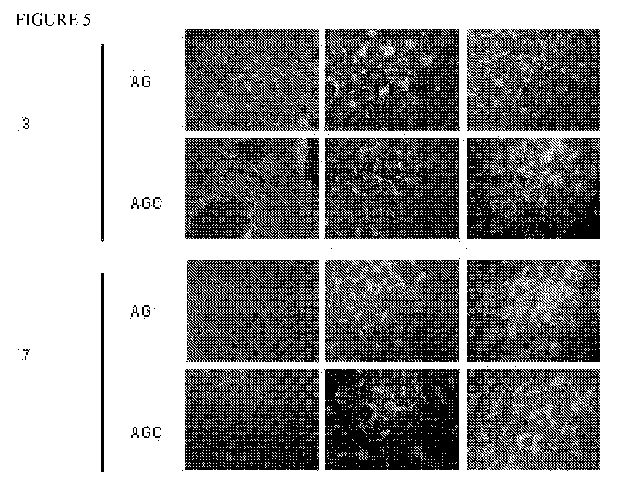

Figure 5. PLA/G5 promotes proliferation, migration and sprouting of rEPCs (rat endothelial progenitor cells). Cells were cultured during 3 and 7 days on angiogenic conditions (AGC⁺, with the presence of a collagen coating) or without (AGC⁻). Cells were stained with acridine orange and observed under a fluorescence microscope.
Figure 6. Tubule quantification. EPCs grown over the composites were tested for tube formation in AGC- and AGC+. PLA/G5 provoked an strong induction in capillary-like formation in both conditions.
Figure 7. Transwell migration assay. When cells are seeded on the upper compartment of a Boyden chamber and PLA/G5 discs are placed in the lower compartment a migratory effect is induced, independently of FBS (fetal bovine serum) activation.
PLA/G5 results were similar to those obtained with VEGF (vascular endothelial growth factor). None of the other two compositions induce this behaviour. We hypothesize cells are attracted by a local ionic gradient produced by glass solubilisation.
Figure 8. Real time RT-PCR for vWF and CD31 demonstrated early differentiation to endothelial cells when in contact with the glass G5 (5% TiO₂). Production of the pro-angiogenic cytokines VEGF and IGF2 was also up-regulated. Cells were seeded for 24 h and 3 days, and the expression of the different genes was evaluated.
Figure 9. Glass films produced by sol-gel technique support EPCs survival and proliferation. Cells were seeded for 3 and 7 days, stained with acridine orange, and observed under fluorescence microscope.
Figure 10. Osteoblasts-like cells on PLA-glass composite scaffolds. Cells adhere preferentially when glass particles are present.
Figure 11. Typical XRD spectrum of a G5 amorphous glass where no crystalline peaks are present.
Figure 12. DSC spectrum of a G5 amorphous glass showing Tg at 541.5°C and Tc at 564.5°C.

### Description of the invention

The present invention relates to a nanostructured material comprising a glass with the formulation P₂O₅:CaO:MgO:Y₂O:TiO₂, wherein Y is K or Na.

The term "nanostructured" relates to an assembled material whose structural features exhibit at least one dimension in the nanometer range.

In one embodiment, the present invention relates to a nanostructured material comprising a glass with the formulation P₂O₅:CaO:MgO:Na₂O:TiO₂.

In another embodiment, the present invention relates to a nanostructured material comprising a glass with the formulation P₂O₅:CaO:MgO:K₂O:TiO₂.

In a preferred embodiment, the nanostructured material comprising a glass with the formulation P₂O₅:CaO:MgO:Y₂O:TiO₂, wherein Y is K or Na, has a molar ratio for each component in the glass equals to 44,5:44,5-x:x:y:11-y, respectively, wherein x is a value equal or higher than 0 and lower than 44,5, and wherein y is a value higher than 0 and lower or equal to 11.

These values "x" and "y" can be any positive integer or decimal number (except for "x" which can also be equal to 0), providing that the previous conditions related to the formula are complied.

In a preferred embodiment, the nanostructured material comprising a glass with the formulation P₂O₅:CaO:MgO:Y₂O:TiO₂, wherein Y is K or Na is in the shape of inorganic nanoparticles, composite nanostructured biodegradable fibers, composite nanostructured scaffolds, injectable composite gel, inorganic films or composite films.

The term "inorganic" refers in the present invention to any compound not containing carbon atoms.

The term "composite" refers in the present invention to a multicomponent material comprising multiple, different (non-gaseous) phase domains in which at least one type of phase domain is a continuous phase.

The term "nanoparticle" refers in the present invention to an inorganic glass particle with dimensions in the nanometer range.

The term "biodegradable" refers in the present invention to those materials which breakdown by the effect of the physiological environment.

The term "fiber" refers in the present invention to continuous filaments or discrete elongated pieces of a particular material.

The term "scaffold" refers in the present invention to a processed biomaterial that act as template and provides the necessary support for cell growth and differentiation in which new tissue can regenerate.

The term "film" refers in the present invention to a material that can be deposited as a thin coating in a desired shape.

In another preferred embodiment, the nanostructured material comprising a glass with the formulation P₂O₅:CaO:MgO:Y₂O:TiO₂, wherein Y is K or Na, has a molar ratio for each component in the glass equals to 44,5:44,5-x:x:y:11-y, respectively, wherein x is a value equal or higher than 0 and lower than 44,5, and wherein y is a value higher than 0 and lower or equal to 11 and said nanostructured material is in the shape of inorganic nanoparticles, composite nanostructured biodegradable fibers, composite nanostructured scaffolds, injectable composite gel, inorganic films or composite films.

In a particular embodiment, said nanostructured material disclosed above with all mentioned embodiments consists only of said glass component, being said particular embodiment also applicable to the process of manufacture, uses and method of treatment disclosed below.

The present invention also relates to a process for the preparation of a nanostructured material comprising a glass with the formulation P₂O₅:CaO:MgO:Y₂O:TiO₂, wherein Y is K or Na, comprising the steps of:
a) Preparing titanium, phosphorous, potassium or sodium, calcium and magnesium precursor solutions;
b) Preparing a glass precursor solution by mixing in an inert atmosphere the titanium precursor solution; the calcium precursor solution; the magnesium precursor solution; the sodium or potassium precursor solution and the phosphorus precursor solution;
   wherein the molar ratio for P:Ca:Mg:Y:Ti is 89:44,5-x:x:2y:11-y, wherein Y is K or Na, and wherein x is a value equal or higher than 0 and lower than 44,5, and wherein y is a value higher than 0 and lower or equal to 11; and
c) Shaping the precursor mix solution obtained in step b)

In a particular embodiment, the present invention also relates to a process for the preparation of a nanostructured material comprising a glass with the formulation P₂O₅:CaO:MgO:Y₂O:TiO₂, wherein Y is K or Na, and a molar ratio for each component in the glass equals to 44,5:44,5-x:x:y:11-y, respectively, wherein x is a value equal or higher than 0 and lower than 44,5, and wherein y is a value higher than 0 and lower or equal to 11.

In a particular embodiment, the present invention also relates to a process for the preparation of a nanostructured material comprising a glass with the formulation P₂O₅:CaO:MgO:Y₂O:TiO₂, wherein Y is K or Na, and said nanostructured material is in the shape of inorganic nanoparticles, composite nanostructured biodegradable fibers, composite nanostructured scaffolds, injectable composite gel, inorganic films or composite films.

In a particular embodiment, the present invention also relates to a process for the preparation of a nanostructured material comprising a glass with the formulation P₂O₅:CaO:MgO:Y₂O:TiO₂, wherein Y is K or Na and a molar ratio for each component in the glass equals to 44,5:44,5-x:x:y:11-y, respectively, wherein x is a value equal or higher than 0 and lower than 44,5, and wherein y is a value higher than 0 and lower or equal to 11, and said nanostructured material is in the shape of inorganic nanoparticles, composite nanostructured biodegradable fibers, composite nanostructured scaffolds, injectable composite gel, inorganic films or composite films.

The process of the present invention is carried out preferably in the presence of an inert atmosphere due to the possible reaction of some reactants with air humidity. For this reason, very dried lab tools are desirable. When using the glass precursor solution, the use of low temperatures (for example, 0-6°C) and vigorous stirring (for example, 800-1600 rpm) when homogenizing the mix in step b) is preferred, in order to avoid a previous reaction of said glass precursor solution with residual water coming from the recipients or from the environment.

In a particular embodiment, the present invention relates to a process for the preparation of a nanostructured material comprising a glass with the formulation P₂O₅:CaO:MgO:Y₂O:TiO₂, wherein in the step a) the precursor solutions are titanium, phosphorous, sodium, calcium and magnesium precursor solutions. Accordingly, the precursor solutions in step b) are also titanium, phosphorous, sodium, calcium and magnesium precursor solutions.

In a particular embodiment, the present invention relates to a process for the preparation of a nanostructured material comprising a glass with the formulation P₂O₅:CaO:MgO:K₂O:TiO₂, wherein in the step a) the precursor solutions are titanium, phosphorous, potassium, calcium and magnesium precursor solutions. Accordingly, the precursor solutions in step b) are also titanium, phosphorous, potassium, calcium and magnesium precursor solutions.

In a preferred embodiment for step a), the titanium precursor solution is prepared in step a) by dissolving a titanium salt in absolute ethanol in an inert atmosphere.

The term "titanium salt" refers in the present invention to any ionic compound or coordination complex which involves one or several titanium cations and several inorganic or organic ligands. The use of titanium alkoxides, titanium β-diketonates and/or titanium β-diketonates-alkoxides is preferred.

The term "inert atmosphere" refers in the present invention to the use of an inert gas which is a non-reactive substance in the gaseous state used during chemical synthesis, chemical analysis, or preservation of reactive materials. The use of highly purified nitrogen or argon gas is preferred.

In a preferred embodiment for step a), the phosphorous precursor solution is prepared by refluxing phosphorus pentoxide or a phosphorus salt in a pure alcohol in an inert atmosphere at a temperature between 60C and the boiling point of the alcohol.

The term "phosphorous salt" refers in the present invention to any ionic compound or coordination complex which involves one or several phosphorus cations and several inorganic or organic ligands. The use of phosphorus alkoxides or the result of refluxing phosphorus pentoxide in absolute ethanol is preferred.

In a preferred embodiment for step a), the calcium precursor solution is prepared by refluxing metallic calcium or a calcium salt with 2-methoxyethanol in an inert atmosphere at a temperature between 60 and 124°C.

The term "calcium salt" refers in the present invention to any ionic compound or coordination complex which involves one or several calcium cations and several inorganic or organic ligands. The use of calcium alkoxides is preferred.

In a preferred embodiment for step a), the magnesium precursor solution is prepared by refluxing metallic magnesium or a magnesium salt with 2-methoxyethanol in an inert atmosphere at a temperature between 60 and 124°C.

The term "magnesium salt" refers in the present invention to any ionic compound or coordination complex which involves one or several magnesium cations and several inorganic or organic ligands. The use of magnesium alkoxides is preferred. In a preferred embodiment for step a), the sodium precursor solution is prepared by refluxing metallic sodium or a sodium salt with 2-methoxyethanol in an inert atmosphere at a temperature between 60 and 124°C.

The term "sodium salt" refers in the present invention to any ionic compound or coordination complex which involves one or several sodium cations and one or several inorganic or organic ligands. The use of sodium alkoxides is preferred.

In a preferred embodiment for step a), the potassium precursor solution is prepared by refluxing metallic potassium or a potassium salt with 2-methoxyethanol in an inert atmosphere at a temperature between 60 and 124°C.

The term "potassium salt" refers in the present invention to any ionic compound or coordination complex which involves one or several potassium cations and one or several inorganic or organic ligands. The use of potassium alkoxides is preferred.

In a preferred embodiment for step c), this is carried out by optionally adding to the precursor mix solution obtained in step b) water (in order to enhance the control of the degree of hydrolysis) and/or a non-polar solvent (in order to enhance the control of the particle size), for obtaining said nanostructured material in the form of inorganic nanoparticles. This material can be cured by heating at a temperature between room temperature and 600°C.

The term "non-polar solvent" refers in the present invention to, but without being limited to, solvents with a dielectric constant of less than 15. These solvents have molecules whose electric charges are equally distributed. Non-limiting examples of a non-polar solvent are chloroform, 1,4-dioxane, tetrahydrofuran.

In a more preferred embodiment, the inorganic nanoparticles obtained in step c) are homogeneously dispersed in a biodegradable hydrogel, biodegradable polymer or a mixture of polymers for obtaining said nanostructured material in the form of injectable composite gel.

The term "biodegradable hydrogel" refers in the present invention to a non-fluid colloidal polymer network that is expanded throughout its whole volume by a fluid, whose chains are cleaved by hydrolysis in the physiological medium, and whose degradation by-products are eliminated from the body through metabolic pathways. Non-limiting examples of biodegradable hydrogel are hydrolyzed gelatine, hydrolyzed chitosan, hydrolyzed polyvinyl alcohol or hydrolyzed hydroxypropyl methyl cellulose.

The term "biodegradable polymer" refers in the present invention to those polymers whose chains are cleaved by hydrolysis in the physiological medium, and whose degradation by-products are eliminated from the body through metabolic pathways. Non-limiting examples of biodegradable polymers are polylactides (PLA), poly(L-lactide)(PLLA), poly(D,L-lactide) (PDLLA), Polycaprolactone, polyglycolides (PGA) and their copolymer: Poly(D,L-lactide-co-glycolide) (PLGA).

In another preferred embodiment for step c), this is carried out by electrospinning method, adding water, one or more biodegradable polymers and a non polar and volatile solvent for obtaining said nanostructured material in the form of composite nanostructured biodegradable fibers.

Briefly, the "electrospinning method" consists of a high voltage electrical charge to an electrically charged jet of polymer solution or melt, which dries or solidifies to leave a polymer fiber (typically on the micro or nano scale). It generally consists of a syringe which is connected to a voltage power source and a collector connected to the earth.

In another preferred embodiment for step c), this is carried out by rapid prototyping, solvent casting-particle leaching or freeze-drying methods, adding water and one or more biodegradable polymers and optionally adding a non-polar solvent and/or porogen for obtaining said nanostructured material in the form of composite nanostructured scaffolds.

Briefly, the "rapid prototyping" method is a computer assisted design technique used to fabricate physical objects with well defined architectures.

Briefly, the "solvent casting-particle leaching" method is a process for the preparation of porous 3D structures with interconnected porosity. Basically, a polymer (or a polymer-particle mixture) is dissolved in an organic solvent together with a porogen (with specific dimensions) which are then homogeneously mixed in the solution. The slurry is cast in a mold and finally porogens are dissolved in water leaving behind empty pores of sizes in the order of the original particles.

Briefly, the "freeze-drying" method is a process for the preparation of porous 3D structures with interconnected porosity. Basically, a polymer or (or a polymer-particle mixture) is dissolved in an organic solvent. The polymer solution is then submitted to a freezing/dehydration process at low temperatures and pressures to sublimate the solvent leaving behind a high porous structure.

The term "porogen" refers in the present invention to any compound or device which is mixed with a continuous phase and leached out in order to form pores.

In another preferred embodiment for step c), this is carried out in order to obtain said nanostructured material in the form of films. There are two possible options.

A first option is by spin-coating or dip-coating methods optionally adding water and heating the shaped material at a temperature between room temperature and 600°C for obtaining said nanostructured material in the form of inorganic films.

Briefly, the "spin-coating" method consists of a method to prepare uniform thin films on flat substrates. Basically, a drop of a polymer solution (or a polymer-particle mixture) is placed on the substrate and then rotated at high speed in order to spread the fluid by centrifugal force an polymerize all over the surface of the substrate.

Briefly, "dip-coating" is a technique to prepare uniform thin films on flat substrates. It refers to the immersion of a substrate into a deposit containing the coating solution and pulling it up from the solution in order to drain it and obtain a thin coating.

A second option is by tape-casting, spin-coating or dip-coating methods optionally adding water and/or one or more biodegradable polymers and/or a non-polar solvent for obtaining said nanostructured material in the form of composite films.

Briefly, the term "tape-casting" refers to a method to prepare uniform thick films on flat substrates. It consists in casting a polymer slurry (or a polymer-particle slurry) containing an organic volatile solvent on a flat substrate, through the use of a smooth blade edge which spreads the slurry to get the suitable thickness. After solvent evaporation a film is obtained.

The present invention also relates to the use of the nanostructured material comprising a glass with the formulation P₂O₅:CaO:MgO:Y₂O:TiO₂, wherein Y is K or Na, as a tuneable control ion release agent with specific target cell signalling.

The present invention also relates to a nanostructured material comprising a glass with the formulation P₂O₅:CaO:MgO:Y₂O:TiO₂, wherein Y is K or Na, for use as a tuneable control ion release agent with specific target cell signalling.

In a particular embodiment, the present invention also relates to the use of a nanostructured material comprising a glass with the formulation P₂O₅:CaO:MgO:Y₂O:TiO₂, wherein Y is K or Na, and a molar ratio for each component in the glass equals to 44,5:44,5-x:x:y:11-y, respectively, wherein x is a value equal or higher than 0 and lower than 44,5, and wherein y is a value higher than 0 and lower or equal to 11, as a tuneable control ion release agent with specific target cell signalling.

In a particular embodiment, the present invention also relates to a nanostructured material comprising a glass with the formulation P₂O₅:CaO:MgO:Y₂O:TiO₂, wherein Y is K or Na, and a molar ratio for each component in the glass equals to 44,5:44,5-x:x:y:11-y, respectively, wherein x is a value equal or higher than 0 and lower than 44,5, and wherein y is a value higher than 0 and lower or equal to 11, for use as a tuneable control ion release agent with specific target cell signalling.

In a particular embodiment, the present invention also relates to the use of a nanostructured material comprising a glass with the formulation P₂O₅:CaO:MgO:Y₂O:TiO₂, wherein Y is K or Na, and said nanostructured material is in the shape of inorganic nanoparticles, composite nanostructured biodegradable fibers, composite nanostructured scaffolds, injectable composite gel, inorganic films or composite films, as a tuneable control ion release agent with specific target cell signalling.

In a particular embodiment, the present invention also relates to a nanostructured material comprising a glass with the formulation P₂O₅:CaO:MgO:Y₂O:TiO₂, wherein Y is K or Na, and said nanostructured material is in the shape of inorganic nanoparticles, composite nanostructured biodegradable fibers, composite nanostructured scaffolds, injectable composite gel, inorganic films or composite films, for use as a tuneable control ion release agent with specific target cell signalling.

In a particular embodiment, the present invention also relates to the use of a nanostructured material comprising a glass with the formulation P₂O₅:CaO:MgO:Y₂O:TiO₂, wherein Y is K or Na and a molar ratio for each component in the glass equals to 44,5:44,5-x:x:y:11-y, respectively, wherein x is a value equal or higher than 0 and lower than 44,5, and wherein y is a value higher than 0 and lower or equal to 11, and said nanostructured material is in the shape of inorganic nanoparticles, composite nanostructured biodegradable fibers, composite nanostructured scaffolds, injectable composite gel, inorganic films or composite films, as a tuneable control ion release agent with specific target cell signalling.

In a particular embodiment, the present invention also relates to a nanostructured material comprising a glass with the formulation P₂O₅:CaO:MgO:Y₂O:TiO₂, wherein Y is K or Na and a molar ratio for each component in the glass equals to 44,5:44,5-x:x:y:11-y, respectively, wherein x is a value equal or higher than 0 and lower than 44,5, and wherein y is a value higher than 0 and lower or equal to 11, and said nanostructured material is in the shape of inorganic nanoparticles, composite nanostructured biodegradable fibers, composite nanostructured scaffolds, injectable composite gel, inorganic films or composite films, for use as a tuneable control ion release agent with specific target cell signalling.

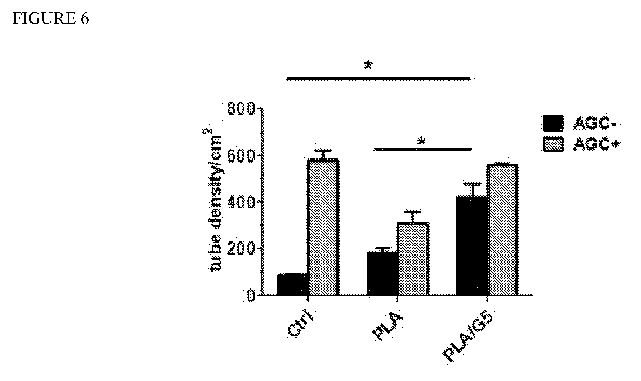

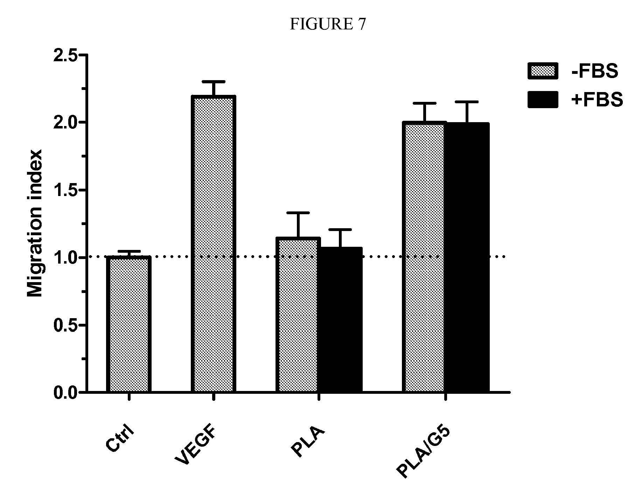

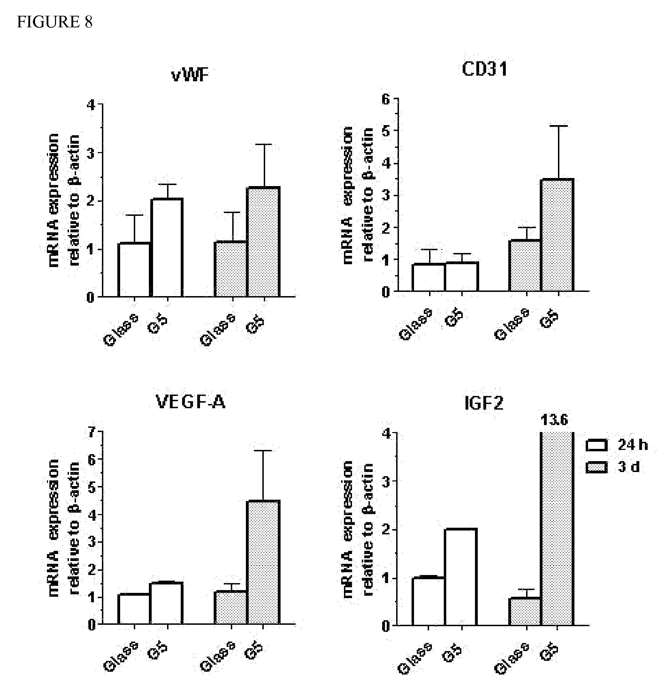

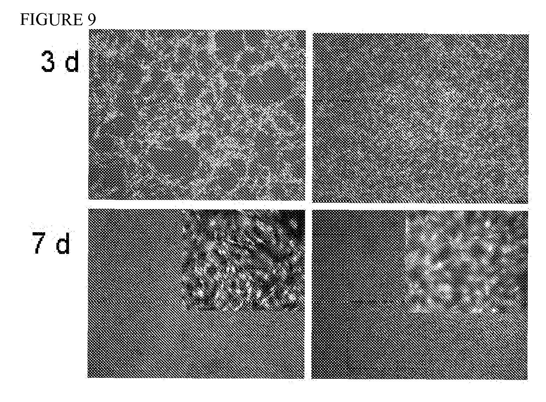

The term "tuneable control ion release agent with specific target cell signalling" refers in the present invention to a material or device which can easily modify its ion release properties by small changes in its structure or composition. The released agents are able to trigger a specific cell response. In this particular case, Ca²⁺ acts as "release agent with specific target cell signalling" (Figures 6-9).

In a preferred embodiment applicable to all the previous use and composition (nanostructured material) for use embodiments, said "tuneable control ion release agent" acts on tissue regeneration. In a more preferred embodiment said tissue is bone tissue or vascular tissue. In an even more preferred embodiment, said vascular tissue is involved in the wound healing process.

Finally, the present invention also relates to a method of treatment comprising the application of the nanostructured material disclosed herein (including all the embodiments enclosed herein) to a subject for regenerating a tissue, with this tissue being preferably bone tissue or vascular tissue. In a preferred embodiment, the present invention relates to a method of treatment comprising the application of the nanostructured material disclosed herein (including all the embodiments enclosed herein) to a subject for healing a wound.

The following examples are included to further illustrate the present invention without being limited thereto.

It should be noted that the nanostructured material described in the present invention in all its embodiments and by way of illustrative purposes in the following examples can be used in any injury, pathology and/or tissue regeneration process, where angiogenesis plays a key role, such as in bone (e.g. pseudarthosis), vascular (e.g. atherosclerosis) and skin (e.g. bums) healing.

### EXAMPLES

### Example 1:

Glass films can be prepared by spin-coating or dip-coating when a borosilicate glass substrate is used. The substrate is attached to the sample holder by using vacuum in the case of spin-coating. Then, a drop of 100µl of glass precursor solution is deposited on the surface. Using a suitable speed (2000-8000 rpm) for 1 minute at room temperature of an organo-metallic gel of several nanometers thickness is achieved. In the case of dip-coating, the substrate is vertically held with a clip and immersed in the glass precursor solution. Then, it is gradually pulled out from the solution at a speed between 50 and 80 mm/minute. An organo-metallic gel film of several nanometers thickness is obtained and dried with the help of an infrared heat lamp. In both cases, the film undergoes a thermal treatment for two hours at a temperature below the crystallization temperature (Tc) of the glass to avoid its crystallization. In the case of the composition P₂O₅:CaO:MgO:Y₂O:TiO₂ 44.5:44.5:0:6:5 (coded G5) this temperature is about 564.5°C. Temperature ramps must be lower than 60°C/hour to avoid the formation of cracks due to the different thermal expansion coefficient of the film and the substrate. At the end of the treatment, an inorganic glass film is obtained.

### Example 2:

Glass nanoparticles can be obtained by evaporation of the solvent of the glass precursor solution using a rotary evaporator or by freeze-drying. In the case of the composition P₂O₅:CaO:MgO:Y₂O:TiO₂ 44.5:44.5:0:6:5, 1200 µl of catalyst of H₂O:NH₃:2-propanol, with the ratio 4:0.3:12, are added to 20ml of the precursor solution. Then, solvent is gradually evaporated using a rotary evaporator until gelation and complete dryness of the gel. The resulting solid is placed in a furnace to be thermally treated at a temperature below the Tc of the glass to avoid its crystallization. In the case of the composition P₂O₅:CaO:MgO:Y₂O:TiO₂ 44.5:44.5:0:6:5 this temperature is about 564.5°C and it is held for 5 hours to have enough time to completely eliminate organic material. Thermal ramps can be faster than in the case of films (300°C/hour). The furnace must be a combustion type to help the organic fumes to exhaust from the system.

### Example 3:

Glass nanoparticles can be obtained using a determined amount of glass precursor solution and the suitable amount of water and acid or base. The mix is aged at 20-60°C for hours, days or months in order to obtain the desired particle size. In this way, the hydrolysis process is favoured and nanometric glass particles are obtained at low temperature. In this sense, 20 ml of precursor solution in the system P₂O₅:CaO:MgO:Y₂O:TiO₂ 44.5:44.5:0:6:5 are hydrolyzed using 1200 µl a catalyst of H₂O:NH₃:2-propanol with the ratio 60:0.3:12. The mix is heated at 60°C during 24 hours applying a vigorous stirring.
The size and shape of the particles can be controlled by inverse micelle method which involves the use of a hydrophobic solvent such as cyclohexane or dioxane and surfactants such as TRITON X-100 to produce micelles which act as a micro or nanoreactors where the particle grows isolated from the rest of the media. In this sense, 20 ml of precursor solution of the system P₂O₅:CaO:MgO:Y₂O:TiO₂ 44.5:44.5:0:6:5 are mixed with 1200 µl a catalyst of H₂O:NH₃:2-propanol with the ratio 60:0.3:12, 60 ml of cyclohexane and 10 ml of TRITON X-100. The mixture is heated at 60°C during 24 hours applying a vigorous stirring. Then the mix is centrifuged at 3000 r.p.m. for 10 minutes, and the precipitate is rinsed with 2-propanol to remove surfactants or, on the other hand, heated in the furnace at a temperature below the Tc.

### Example 4:

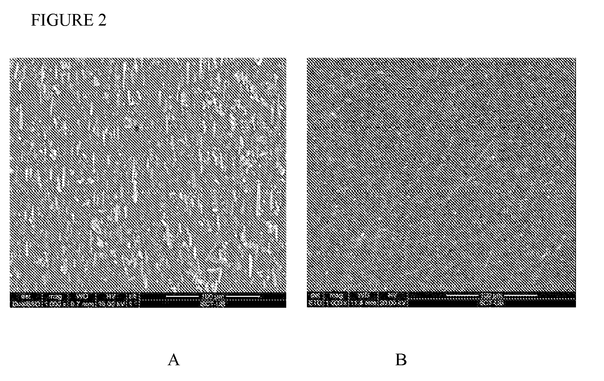

A composite material can be obtained by adding 1200 µl of a catalyst of H₂O:NH₃:2-propanol with the ratio 60:0.3:12 to 20 ml of glass precursor solution of the system P₂O₅:CaO:MgO:Y₂O:TiO₂ 44.5:44.5:0:6:5. Immediately, 60 ml of 1,4-dioxane are added. The mix is heated at 60°C during 24 hours applying a vigorous stirring. The colour of the mix will vary from clear yellow to completely white. At this point, 2g of poly(lactic) (PDLLA) acid can be added and dissolved. It usually takes around 20 minutes. At this stage the composite material precursor slurry is obtained. The slurry is finally evaporated at air to obtain a composite slide (figure 2).

### Example 5:

Composite material described in example 4 is suitable to be deposited by electrospinning in order to produce 2D scaffolds for tissue engineering and regenerative medicine. The composite material precursor slurry obtained in example 4 contains glass nanoparticles homogeneously dispersed in an organic solvent with a biodegradable polymer which acts as binder. The viscosity of the slurry must to be adjusted using 1,4-dioxane to obtain a viscosity ranging from 400 to 1000 cps. Thus, the slurry with a suitable viscosity can be easily injected using a syringe with a tip (gauge = 12.7 mm), which is held in an infusion pump applying a flow rate of 1 ml/hour and connected to earth. A flat collector also connected to earth is placed in front of the syringe tip at a distance of 150 mm. The tip is connected to a power source and a voltage of 8KV is applied. A thin jet will appear forming a Taylor cone which will travel from the tip to the collector forming a non-woven melange of composite fibers.

### Example 6:

Composite material described in example 4 is suitable to be deposited by rapid prototyping in order to produce 3D scaffolds for tissue engineering and regenerative medicine. The composite material precursor slurry obtained in example 4 contains glass nanoparticles homogeneously dispersed in an organic solvent with a biodegradable polymer which acts as binder. The viscosity of the slurry must be adjusted using chloroform to obtain a viscosity ranging from 500 to 2000cps. This slurry can be deposited in a layer-by-layer way using a syringe with a tip (gauge range=10-20mm) which is connected to the smart pump system of the instrument. Processing conditions namely pressure (10 psi) and motor speed (8mm/s) can be tuned through the instrument software; processing temperature (60°C) can be set using an external heating device. Finally, a 3D structure mimicking the specific design made with the instrument software is obtained.

### Example 7:

Particles obtained by the methods described in example 2 and 3 are suitable to be injected ultrasonically dispersed in a hydrogel such as hydrolyzed gelatine, hydrolyzed chitosan, hydrolyzed polyvinyl alcohol or hydrolyzed hydroxypropyl methyl cellulose, or polymer such as polylactides (PLA), poly(L-lactide)(PLLA), poly(D,L-lactide) (PDLLA), Polycaprolactone, polyglycolides (PGA) and their copolymer: Poly(D,L-lactide-co-glycolide) (PLGA). 1 g of nano particles can be ultrasonically dispersed in hydrolized gelatine to obtain a slurry of a viscosity between 1000 and 2000 cps. The viscosity can be adjusted with water. A 5 ml syringe can be filled with the resulting slurry. An injectable biodegradable material is finally obtained.

### Example 8

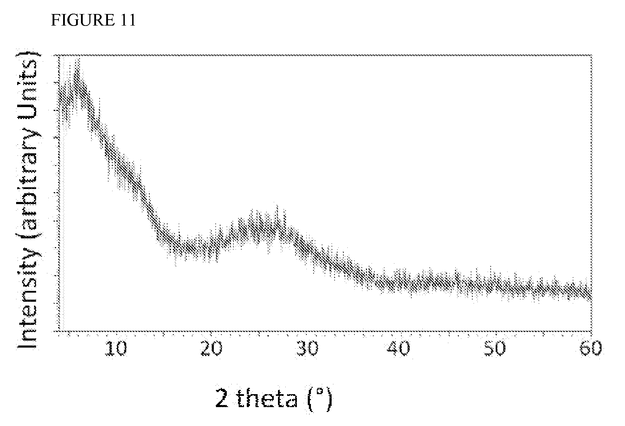

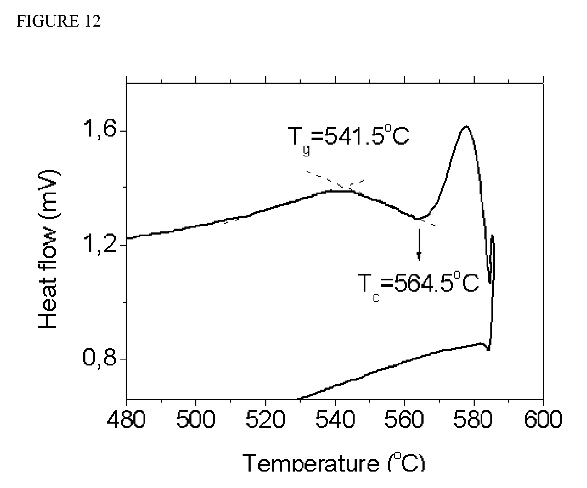

A typical characterization of glasses involves: differential scanning calorimetry (DSC) to get glass transition temperature (Tg) which appears as a small step just before crystallization temperature (Tc) (figure 12): X-ray diffraction (XRD) for evaluating amorphicity and check optimum purity of the glass, it is shown in the form of a broad and short peak (figure 11); scanning electron microscopy coupled with energy dispersive spectroscopy (SEM-EDS) to evaluate the morphology and stoichiometry of the glass; electron micro probe analysis (EMPA), that must show an experimental ionic distribution similar to the one theoretically proposed. Other characterization techniques usually used for these glasses are Z potential, which is usually negative, and contact angle measurement (which is usually low) to evaluate surface properties. Other techniques for evaluating the ion release features are measurements by ion selective electrode (ISE) and inductively coupled plasma spectrometry (ICP-OES or ICP-MS) along time immersed in an aqueous solution (figure 4). For a typical inorganic phosphate glass in the system P₂O₅:CaO:MgO:Y₂O:TiO₂ 44.5:44.5:0:6:5 fabricated at 532.9°C, no peaks were observed in the XRD spectrum (figure 11). DSC showed a glass transition temperature of 541.5°C while SEM-EDS showed a composition of P₂O₅:CaO:MgO:Y₂O:TiO₂ 43.7±0.7 : 47.2±0.5 : 4.4±0.5 : 4.7±0.3. The measure of Z potential showed a value of -76.5±1.2 mV and a water contact angle of 29.9 ± 1°. Measurements of the ion release properties in simulated body fluid (SBF) carried out by ICP-MS showed that there is a strong release of Ca²⁺, Na⁺, HₓPO₄^{3-x} and Ti⁴⁺ during the first 3 days. ISE confirmed this behaviour and furthermore it showed that pH of the medium decreases until nearly 7 during the first 3 days. Then, it reaches the usual pH of 7.4.

### References

1.- Brow RK. Review: the structure of simple phosphate glasses. Journal of Non-Crystalline Solids. 2000; 263 (1-4):1-28
2.- Carta D, Pickup D, Knowles JC, Smith ME, Newport RJ. Sol-gel synthesis of the P2O5-CaO-Na2O-SiO2 system as a novel bioresorbable glass, Journal of Materials Chemistry, 2005; 15: 2134-2140.
3.- Knowles J. Phosphate based glasses for biomedical applications. Journal of Materials Chemistry 2003; 13:2395-2401.
4.- Navarro M, Ginebra MP, Clement J, Martinez S. Avila G. Planell JA. Physicochemical degradation of titania-stabilized soluble phosphate glasses for medical applications. Journal of the American Ceramic Society 2003; 86(8): 1345-1352.
5.- Navarro M, Ginebra MP, Planell JA. Cellular response to calcium phosphate glasses with controlled solubility. Journal of Biomedical Materials Research 2003a; 67A: 1009-1015.
6.- David M. Pickup, Kate M. Wetherall, Jonathan C. Knowles, Mark E.Smith, Robert J. Newport Sol-gel preparation and high-energy XRD study of (CaO)x(TiO2)0.5-x(P2O5)0.5 glasses (x = 0 and 0.25)J Mater Sci: Mater Med 2008; (19):1661-1668
7.- Zachariesen WH. The atomic arrangement in glass: Journal of the American Ceramic Society. 1932; 54 (10): 3841-3851.
8.- V. Salih, K. Franks, M. James, G. W. Hastings, J. C. Knowles and I. Olsen. Development of soluble glasses for biomedical use Part II: The biological response of human osteoblast cell lines to phosphate-based soluble glasses. J Mater Sci: Mater Med 2000; (11):615-620
9.- ASTM Standard C162, 2005, "Standard Terminology of Glass and Glass Products" ASTM International, West Conhohocken, PA, 2005, DOI: 10.1520/C0162-05. www.astm.org

## Claims

1. Nanostructured material comprising a glass with the formulation P₂O₅:CaO:MgO:Y₂O:TiO₂,
wherein Y is K or Na.

2. Nanostructured material according to claim 1, wherein the molar ratio of each component in the glass is 44,5:44,5-x:x:y: 11-y, respectively, wherein x is a value equal or higher than 0 and lower than 44,5, and wherein y is a value higher than 0 and lower or equal to 11.

3. Nanostructured material according to any of the preceding claims in the shape of inorganic nanoparticles, composite nanostructured biodegradable fibers, composite nanostructured scaffolds, injectable composite gel, inorganic films or composite films.

4. Process for the preparation of a nanostructured material according to any of claims 1 to 3 comprising the steps of:
a) Preparing titanium, phosphorous, potassium or sodium, calcium and magnesium precursor solutions;
b) Preparing a glass precursor solution by mixing in an inert atmosphere the titanium precursor solution; the calcium precursor solution; the magnesium precursor solution; the sodium or potassium precursor solution and the phosphorus precursor solution ;
wherein the molar ratio for P:Ca:Mg:Y:Ti is 89:44,5-x:x:2y:11-y, wherein Y is K or Na, and wherein x is a value equal or higher than 0 and lower than 44,5, and wherein y is a value higher than 0 and lower or equal to 11; and
c) Shaping the glass precursor solution obtained in step b)

5. Process for the preparation of a nanostructured material according to claim 4 wherein the step c) is carried out by optionally adding water and/or a non polar-solvent for obtaining said nanostructured material in the form of inorganic nanoparticles, heating then the shaped material at a temperature between room temperature and 600°C.

6. Process according to claim 5, wherein the inorganic nanoparticles obtained are homogeneously dispersed in a biodegradable hydrogel, biodegradable polymer or a mixture of polymers for obtaining said nanostructured material in the form of injectable composite gel.

7. Process for the preparation of a nanostructured material according to claim 4 wherein the step c) is carried out by electrospinning method, adding water, one or more biodegradable polymers and a non polar and volatile solvent for obtaining said nanostructured material in the form of composite nanostructured biodegradable fibers.

8. Process for the preparation of a nanostructured material according to claim 4 wherein the step c) is carried out by rapid prototyping, solvent casting-particle leaching or freeze-drying methods, adding water and one or more biodegradable polymers and optionally adding a non-polar solvent and/or porogen for obtaining said nanostructured material in the form of composite nanostructured scaffolds.

9. Process for the preparation of a nanostructured material according to claim 4 in the form of films, wherein the step c) is carried out by spin-coating or dip-coating methods optionally adding water and heating the shaped material at a temperature between room temperature and 600°C for obtaining said nanostructured material in the form of inorganic films or the step c) is carried out by tape-casting, spin-coating or dip-coating methods optionally adding water and/or one or more biodegradable polymers and/or a non-polar solvent for obtaining said nanostructured material in the form of composite films.

10. Process according to any of claims 4 to 9, wherein the titanium precursor solution is prepared by dissolving a titanium salt in absolute ethanol.

11. Process according to any of claims 4 to 9, wherein the phosphorous precursor solution is prepared by refluxing a phosphorous pentoxide or a phosphorus salt in a pure alcohol in an inert atmosphere at a temperature between 60 and the boiling point of the alcohol.

12. Process according to any of claims 4 to 9, wherein the calcium and magnesium precursor solutions are prepared by refluxing metallic calcium or magnesium or a calcium or magnesium salt, respectively, with 2-methoxyethanol in an inert atmosphere at a temperature between 60 and 124°C.

13. Process according to any of claims 4 to 9, wherein the sodium or potassium precursor solution is prepared by refluxing metallic sodium or potassium or a sodium or potassium salt, respectively, with 2-methoxyethanol in an inert atmosphere at a temperature between 60 and 124°C.

14. Use of the nanostructured material according to any of claims 1 to 3 as a tuneable control ion release agent with specific target cell signalling.

15. Use according to claim 14 for tissue regeneration.

16. Use according to claim 15, wherein the tissue is bone tissue or vascular tissue.

17. Use according to claim 16, wherein the vascular tissue is involved in the wound healing process.
